(19) Europäisches Patentamt

European Patent Office

Office européen des brevets

(11) Publication number: **0 558 148 A1**

(12) **EUROPEAN PATENT APPLICATION**

(21) Application number: 93200528.3

(22) Date of filing: **24.02.93**

(51) Int. Cl.5: **C07B 35/04**, C07C 5/42, B01J 23/00

(30) Priority: **26.02.92 EP 92200572**

(43) Date of publication of application:
**01.09.93 Bulletin 93/35**

(84) Designated Contracting States:
**BE DE FR GB IT NL**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Dejaifve, Pierre Eugene Jean**
**Badhuisweg 3**
**NL-1031 CM Amsterdam(NL)**
Inventor: **Lednor, Peter William**
**Badhuisweg 3**
**NL-1031 CM Amsterdam(NL)**
Inventor: **Tromp, Petrus Joannes Josephus**
**Badhuisweg 3**
**NL-1031 CM Amsterdam(NL)**

(54) Process for the dehydrogenation of organic compounds.

(57) A process for the dehydrogenation of organic compounds by contacting a feed containing one or more organic compounds at an elevated temperature with a dehydrogenation catalyst and a perovskite-type oxide which is based upon an oxide of a first metal selected from the Groups 4b, 5b, 6b, 7b, 8 and 1b of the Periodic Table and an oxide of a second metal selected from Group 3b of the Periodic Table and said composition of a dehydrogenation catalyst and perovskite-type oxide.

EP 0 558 148 A1

This invention relates to a process for the dehydrogenation of organic compounds by contacting a feed containing one or more organic compounds at an elevated temperature with a dehydrogenation catalyst.

Processes of this kind are well known for a long time. By these processes the organic compounds can be dehydrogenated, e.g. alkanes to alkenes, with concomitant formation of hydrogen. There are several problems associated with these processes. For example, due to the chemical equilibrium between hydrocarbons, their olefinic dehydrogenation product(s) and hydrogen, it is in general not possible to convert the hydrocarbons completely into the desired olefin(s) so that the yield of olefins is undesirably low.

In several prior art processes molecular oxygen is added to the dehydrogenation feed in order to oxidize the hydrogen which is produced in the dehydrogenation, so that it is possible to operate the process at a conversion level beyond the conversion level at which the concentrations of the reactants are at equilibrium. However, besides oxidation of the hydrogen, the presence of oxygen may also effect oxidation of the organic compounds present with formation of carbon dioxide and carbon monoxide. The gain in conversion may thus be offset by a loss of material due to oxidation.

It is known that certain perovskites which are based upon an oxide of a transition metal are reducible with hydrogen at elevated temperature. It has now unexpectedly been found that it is possible to carry out the dehydrogenation beyond the equilibrium conversion by dehydrogenating in the presence of such a perovskite so that an improved yield of dehydrogenation products can be accomplished.

Accordingly, the present invention relates to a process for the dehydrogenation of organic compounds by contacting a feed containing one or more organic compounds at an elevated temperature with a dehydrogenation catalyst and a perovskite-type oxide which is based upon an oxide of a first metal selected from the Groups 4b, 5b, 6b, 7b, 8 and 1b of the Periodic Table and an oxide of a second metal selected from Group 3b of the Periodic Table.

In addition the invention relates to a catalyst composition for the dehydrogenation of organic compounds comprising a dehydrogenation catalyst and a perovskite-type oxide which is based upon an oxide of a first metal selected from the Groups 4b, 5b, 6b 7b, 8 and 1b of the Periodic Table and an oxide of a second metal selected from Group 3b of the Periodic Table.

Perovskite-type compounds are well known in the art. They possess a crystal structure which is equal to, or relates closely to the crystal structure of the mineral Perovskite (cf. L.G. Tejuca et al., in Advances in Catalysis, Volume 36, page 237 ff.). Perovskite-type oxides are based upon oxides of at least two metals, designated herein as a first metal and a second metal.

In the process of the invention hydrogen which is formed upon dehydrogenation of the organic compound(s) is oxidized by the perovskite-type oxide with high selectively, i.e. virtually without oxidation of the organic compound(s) present in the mixture. The present finding of the highly selective oxidation of hydrogen in the presence of organic compounds, such as alkanes and alkenes, is unexpected as it is known for a long time that the perovskite-type oxides can also be active in the oxidation of organic compounds, viz. they can be used as oxidation catalysts for a large variety of hydrocarbons. The finding is in particular surprising in cases where the organic compounds present are branched alkanes, such as isobutane, and the dehydrogenation products derived therefrom, which are tertiary olefins, such as isobutene. These hydrocarbons are in particular sensitive towards oxidation because of the presence of one or more tertiary hydrogen atoms in branched alkanes and the presence of a relatively large number of allylic hydrogen atoms in tertiary olefins. The present finding is also surprising with a view to the recently discovered fact that when a physical mixture of the metal oxides constituting the perovskite-type oxide is used as the oxidizing agent, rather than the perovskite itself, the selectivity of the hydrogen oxidation is substantially lower, viz. a substantially greater part of the organic compounds is oxidized, as well.

The perovskite-type oxides which can be used in the process of the invention are based upon an oxide of a metal selected from the Groups 4b, 5b, 6b, 7b, 8 and 1b of the Periodic Table, which metal is designated herein as the first metal. Examples of such metals are ruthenium and rhodium. It is preferred to select the first metal from those metals which have an atomic number of at least 26. The most preferred first metal is copper.

The perovskite-type oxides of the present process contain as the second metal a metal selected from Group 3b of the Periodic Table. Preferably the second metal is yttrium. The perovskite-type oxides preferably contain a third metal selected from Group 2a of the Periodic Table. Typical third metals are calcium, strontium and barium.

Particularly good results can be obtained with perovskite-type oxides which are selected from the group consisting of $YBa_2Cu_3O_{7-x}$, $Pb_2Sr_2YCu_3O_8$ and $Pb_2Sr_2Ca_{0.5}Y_{0.5}Cu_3O_8$, wherein x is a number from 0 to 1.

The preparation of the perovskite-type oxides which can be used in the present process is well documented in the art. They can be prepared by grinding, mixing and heating the constituent oxides. It is also possible to apply solution methods, which methods may provide more intimate mixtures of metal

2

containing components prior to formation of a perovskite-type structure by heating. A suitable solution method involves the formation of an amorphous precipitate or a gel from the nitrates of the metals in aqueous citric or oxalic acid, and drying and calcining the precipitate or the gel. Galcining is typically performed in an oxygen-containing gas at a temperature above 800 °C. The perovskite-type oxide may be brought into any suitable shape prior to or after the calcining, using any eligible technique available in the art.

The dehydrogenation catalyst of the invented process is well known in the art and may be of any available type. It will be clear that the dehydrogenation catalyst is different from the perovskite-type oxide.

Typically, the dehydrogenation catalyst is a solid material containing a transition metal and/or a noble metal. More typically the dehydrogenation catalyst comprises one or more metals selected from Groups 5b, 6b, 7b and 8 of the Periodic Table, dispersed on a refractory support. Suitably, the metals of the dehydrogenation catalyst are selected from the group consisting of vanadium, chromium, molybdenum and the Group 8 noble metals, such as platinum and palladium. The refractory support may be of any available type, such as supports comprising magnesium oxide, titanium oxide, magnesium aluminate or aluminium phosphate. Preferred refractory supports comprise silica and in particular alumina, more in particular gamma alumina. Very suitable supports have a porous structure and have a surface area ranging from 10 $m^2/g$ to 500 $m^2/g$, preferably from 50 $m^2/g$ to 400 $m^2/g$.

Preferably the dehydrogenation catalyst contains from 1 to 25 %w and, in particular, from 5 to 20 %w of the Group 5b, 6b, 7b and/or 8 metal(s), relative to the weight of the catalyst. Very good results can be obtained with a chromia-on-alumina catalyst containing 10 %w chromium, 2 %w tin and 2 %w cesium on a gamma alumina support.

The dehydrogenation catalyst may be prepared according to methods well known in the art, for example by impregnating the refractory support with an aqueous solution containing the desired quantity of the transition and/or noble metal and, optionally, the other elements referred to hereinbefore. After the impregnation, the refractory support is suitably dried and calcined, preferably by heating in an oxygen-containing atmosphere at a temperature in the range of from 300 to 800 °c. The catalyst may be brought into any suitable shape prior to the impregnation, or after the impregnation by any suitable technique available in the art.

Before using the catalyst in the process of the invention, the catalyst may be subjected to heat, preferably at the same or approximately the same temperature as the temperature employed in the dehydrogenation process, in the presence of an inert gas, such as argon or nitrogen, or in the presence of a reducing gas, such as hydrogen. A freshly prepared catalyst may be subjected to such a heat treatment, as well as a catalyst which is obtained from a regeneration step, as described hereinafter.

In the process of the invention the dehydrogenation catalyst and the perovskite-type oxide are contacted with a feed containing one or more dehydrogenable organic compounds, such as hydrocarbons and secondary alcohols. Dehydrogenable organic compounds typically contain at least two atoms to each of which one or more hydrogen atoms are attached and upon loss of hydrogen at least two of these atoms become part of a cyclic structure and/or an unsaturated moiety. The unsaturated moiety can be e.g. aromatic, olefinic or ketonic. The feed typically comprises one or more hydrocarbons, more typically one or more linear or branched alkanes, preferably $C_2$ - $C_{10}$ alkanes, more preferably $C_2$ - $C_5$ alkanes. A particularly preferred feed contains propane and/or isobutane. Inert gases, such as nitrogen or helium, may be present in the feed. It is preferred to perform the process of the invention in the substantial absence of oxygen.

The process of the present invention can be carried out using a wide range of operating conditions. The process is suitably carried out in a continuous mode of operation, wherein the feed is continuously led over one or more beds of solid particles of the dehydrogenation catalyst and the perovskite-type oxide. In one mode of operation the particles of the catalyst and the particles of the perovskite-type oxide are mixed and they are typically contained in a single bed of particles. In another mode of operation the particles of the catalyst and the particles of the perovskite-type are not mixed and they are contained in separate beds. In this mode of operation it is preferred to have the two types of beds arranged in an alternating order. The beds may be positioned in one reactor, or they may be distributed over at least two reactors. The beds may be fixed beds or moving beds, such as fluidized beds.

In the present process the dehydrogenation catalyst is suitably operated at a temperature of at least 400 °C, in particular at a temperature in the range of from 500 to 750 °C, more in particular from 550 to 700 °C. It is preferred that the feed is contacted with the catalyst during from 0.01 to 10 seconds, more preferred during from 0.02 to 5 seconds. The perovskite-type oxide is suitably present at a temperature of at least 250 °c, preferably in the range of from 300 to 750 °C and more preferably in the range of from 400 to 700 °c. The contacting time of the feed with the perovskite-type oxide is preferably in the range of from 0.01 to 10 seconds, more preferably in the range of from 0.02 to 5 seconds.

The pressure in the present process can be varied within wide ranges. Suitably the pressure is such that at the prevailing temperature the feedstock is substantially in its gaseous phase. The pressure is preferably in the range of from 0.1 to 5 bar. This can be advantageous since no expensive compressors are necessary.

It will be understood that by the reaction with hydrogen the perovskite-type oxide will be converted to reduced species which may not be active as an oxidation agent. Hence, the quantity of the perovskite-type oxide which may be employed depends on the quantity of hydrogen which can be involved in the dehydrogenation of the particular quantity of organic compound which one wishes to dehydrogenate. Without wishing to be bound to theory it is believed that the oxygen of the perovskite-type oxide which can be considered to be bound to the first metal may be the oxygen which is active in the oxidation of hydrogen, in which oxidation the first metal may be reduced to zero-valent metal. Such a quantity of oxygen bound to the first metal is suitably present in the perovskite-type oxide which is equivalent to at least 0.2 times the quantity of hydrogen involved in the dehydrogenation, preferably from 0.5 to 5 times and more preferably from 0.8 to 1.5 times that quantity of hydrogen.

The reduced species obtained from the perovskite-type oxide after the contacting with the feed can be re-oxidised to obtain a perovskite-type oxide. It has surprisingly been found that this re-oxidized perovskite-type oxide can be used in a subsequent dehydrogenation process with substantial retention of the oxidation capacity and of the selectivity in the oxidation of hydrogen as found in the dehydrogenation process in which the perovskite-type oxide was used before the re-oxidation. The re-oxidation can suitably be effected by a treatment with molecular oxygen containing gas, such as air. Preferably the re-oxidation is carried out at a temperature above 500 °C, in particular at a temperature in the range of from 600 to 1100 °C, more in particular from 750 to 1000 °C. The reaction time in the re-oxidation is typically at least 1 minute, preferably in the range of from 1 to 6 hours, in particular from 2 to 4 hours. The quantity of oxygen to be reacted with the reduced species in the re-oxidation is preferably equal or substantially equal to the quantity of oxygen which had been removed from the perovskite-type oxide in the preceding dehydrogenation by the reaction with hydrogen. Other quantities of oxygen are also possible. Typically the quantity of oxygen to be reacted is at least 0.2 times, more typically at least 0.5 times the quantity of oxygen removed. Without wishing to be bound by theory, the maximum quantity of oxygen which can be reacted is the quantity which may bring the first metal into its highest possible oxidation state (valency). It is clear that the perovskite-type oxide can be re-used many times in the dehydrogenation process of the present invention.

During the dehydrogenation the activity of the dehydrogenation catalyst may decline, for example due to formation of coke. It may be advantageous to regenerate the catalyst after having been contacted with the feed in a regeneration step, preferably, by subjecting the catalyst to a treatment with an oxidizing gas, such as air. It may be advantageous to carry out the catalyst regeneration and the re-oxidation of the reduced species obtained from the perovskite-type oxide in a single step, thus avoiding the need of a solids separation when a mixed bed of the dehydrogenation catalyst and the perovskite-type oxide is employed.

The dehydrogenation products obtained in the present process may be recovered and purified by any suitable technique, such as distillation. Unconverted starting material(s) may be isolated as well and, if desired, fed to a subsequent dehydrogenation, optionally, after combining with fresh feed.

The oxidation of hydrogen by perovskite-type oxides in the presence of organic compounds will now be further illustrated by the Experiments following hereinafter. The present invention will be further illustrated by means of the subsequent Example.

## EXPERIMENTS 1-3

### Oxidation of hydrogen in the presence of isobutene

A quartz tubular reactor (5 mm diameter) was charged with a sample of a copper-containing test material, mixed with 1 g of quartz particles. The bed of particles thus formed was heated at 550 °C in a flow of 0.6 Nl argon/h. At certain intervals of time the flow of argon was interrupted by feeding 2.0 Nml pulses of a mixture of hydrogen, isobutene and helium (45/45/10 by volume) at the same flow rate as the argon flow. From the composition of the gases leaving the reactor, as determined by gas-liquid chromatography, the conversions of hydrogen and isobutene were calculated. The residence time of the gas in contact with the bed was estimated to be approximately 1.0 s, taking into account the temperature, the average pressure of the reaction mixture in the reactor (about 1.1 bar) and the porosity of the bed (about 0.4).

The copper-containing materials tested were 0.2 g of $YBa_2Cu_3O_{6.5}$, 0.2 g of a physical mixture of $Y_2O_3$, BaO and CuO (atomic ratio Y:Ba:Cu 1:2:3) and 0.071 g of CuO (Experiments 1, 2 and 3, respectively). Each sample of test material contained about 0.9 mg atom of oxygen bound to copper. The materials used were commercial samples.

In Experiment 1 it was seen that there was a highly selective hydrogen oxidation for about 20 pulses. Thereafter the hydrogen oxidation ceased because the oxidation capacity of the $YBa_2Cu_3O_{6.5}$ became depleted.

In Experiment 2 it was seen that along with the oxidation of hydrogen there was considerable oxidation of isobutene. The oxygen capacity of the test material became depleted after about 8 pulses. Similar results were obtained in Experiment 3.

The results have been further specified in Table 1.

TABLE 1

| Experiment | Test material | Pulses [1] | Consumption ($\mu$mol)[2] | | Selectivity (%) [3] |
|---|---|---|---|---|---|
| | | | hydrogen | isobutene | |
| 1 | $YBa_2Cu_3O_{6.5}$ | 20 | 810 | 14 | 83 |
| 2[4] | $Y_2O_3/BaO/CuO$ | 8 | 280 | 77 | 23 |
| 3[4] | CuO | 8 | 170 | 69 | 17 |

[1] Number of pulses until depletion of oxygen in test material.

[2] Total consumption during pulses until oxygen depletion.

[3] Defined as 100 x (moles oxygen used in $H_2$ oxidation)/(moles oxygen used in hydrogen and isobutene oxidation), assuming total oxidation of isobutene to $CO_2$ and $H_2O$.

[4] For comparison, test material not according to the invention.

EXPERIMENTS 4-7

Oxidation of hydrogen in the presence of isobutane and isobutene

The procedures of Experiment 1 were repeated, except that the temperature was 600 °C instead of 550 °C and that the following copper-containing materials were tested: 0.2 g of $YBa_2Cu_3O_{6.5}$ (Experiment 4), 0.3 g of $Pb_2Sr_2YCu_3O_8$ (Experiment 5) and 0.3 g of $Pb_2Sr_2Ca_{0.5}Y_{0.5}Cu_3O_8$ (Experiments 6 and 7). The samples of $Pb_2Sr_2YCu_3O_8$ and $Pb_2Sr_2Ca_{0.5}Y_{0.5}Cu_3O_8$ were prepared following the method disclosed by K. Kadowaki et al. in Physica C159 (1989) pp. 165 - 172. In Experiment 7 a mixture of isobutane, isobutene and hydrogen (33/33/33 by volume) was fed instead of a mixture of isobutene, hydrogen and helium. The results are detailed in Table 2.

TABLE 2

| Experiment | Test material | Consumption ($\mu$mol)[1] | | | Selectivity (%)[2] |
|---|---|---|---|---|---|
| | | hydrogen | isobutane | isobutene | |
| 4 | $YBa_2Cu_3O_{6.5}$ | 560 | - | 38 | 49 |
| 5 | $Pb_2Sr_2YCu_3O_8$ | 925 | - | 13 | 85 |
| 6 | $Pb_2Sr_2Ca_{0.5}Y_{0.5}Cu_3O_8$ | 1310 | - | 8 | 93 |
| 7 | $Pb_2Sr_2Ca_{0.5}Y_{0.5}Cu_3O_8$ | 1100 | [3] | [3] | |

[1] Total consumption during pulses until oxygen depletion of test material.

[2] Defined as 100 x (moles oxygen used in $H_2$ oxidation)/(moles oxygen used in $H_2$ and $C_4H_8$ ($C_4H_{10}$) oxidation), assuming total oxidation of hydrocarbons to $CO_2$ and $H_2O$.

[3] Not determined quantitatively; isobutane consumption was lower than isobutene consumption.

EXPERIMENTS 8 - 10

Oxidation of hydrogen in the presence of propane and propene

A quartz tubular reactor (5 mm diameter) was charged with a sample of 1 g of $YBa_2Cu_3O_{6.5}$ particles. The bed of particles thus formed was heated at 500 °C. A flow of 10 Nl/h of a mixture of hydrogen, propane and helium (10/10/80 by volume) (Experiments 8 and 9) or hydrogen, propene and helium (10/10/80 by volume) (Experiment 10) was passed through the reactor. From the composition of the gases leaving the reactor, as determined by gas-liquid chromatography, the conversions of hydrogen and propane or propene were calculated.

In Experiments 8 and 10 the perovskite-type oxides tested were fresh, purchased samples of $YBa_2Cu_3O_{6.5}$, whereas in Experiment 9 a perovskite-type oxide was tested which was obtained by re-oxidation of $YBa_2Cu_3O_{6.5}$ of which the oxygen was depleted in a previous hydrogen oxidation experiment. Prior to use in Experiment 9 this sample was re-oxidized by treating it in a flow of 10 Nml/h of a mixture containing oxygen and helium (5/95 by volume) at 780 °C during 2.5 hour. The results have been recorded in Table 3.

TABLE 3

| Experiment | Perovskite-type oxide | Feed | Consumption($\mu$mol)[2]) | | | Selectivity (%)[2]) |
|---|---|---|---|---|---|---|
| | | | hydrogen | propane | propene | |
| 8 | Fresh | $H_2/C_3H_8$ | 4403 | 13 | - | 97 |
| 9 | Re-oxidized | $H_2/C_3H_8$ | 4470 | 90 | - | 83 |
| 10 | Fresh | $H_2/C_3H_6$ | 5550 | - | 80 | 89 |

[1]) Until depletion of oxygen in the sample of perovskite-type oxide.

[2]) Defined as 100 x (moles oxygen used in $H_2$ oxidation)/(moles oxygen used in $H_2$ and $C_3H_8$ or $C_3H_6$ oxidation), assuming total oxidation of hydrocarbons to $CO_2$ and $H_2O$.

EXAMPLE

Preparation of dehydrogenation catalyst

A chromia-on-alumina catalyst containing 10 %w chromium, 2 %w tin and 2 %w cesium on a gamma alumina support was prepared by pore-volume impregnating the support with an aqueous solution containing $CrO_3$, $CsNO_3$ and $SnSO_4$, followed by drying and calcination (450 °C, 1 h).

Subsequently the catalyst was reduced in a flow of a mixture of hydrogen and argon (20/80 by volume) at 500 °C for 1 h.

Dehydrogenation of isobutane

A quartz tubular reactor (5 mm diameter) was charged with a 500 mg sample of the chromium-on-alumina catalyst and in series herewith (downstream) a physical mixture of 250 mg of the same chromium-on-alumina catalyst and 400 mg of a purchased sample of $YBa_2Cu_3O_6$. The bed of particles thus formed was heated at 550 °C in a flow of 0.2 Nl argon/h, corresponding with a gas residence time in the chromium-on-alumina bed of about 2 s and a gas residence time of about 1 s in the downstream mixed particles bed. At certain intervals of time the flow of argon was interrupted by feeding 2 Nml pulses of a mixture of isobutane and helium (95/5 by volume) at the same flow rate as the argon flow. From the composition of the gases leaving the reactor, as measured by gas-liquid chromatography, the conversion of isobutane and the selectivity and the yield of isobutene in each of the pulses were estimated.

Before depletion of oxygen of $YBa_2Cu_3O_6$ (i.e. up to the thirties pulse; depletion occurred during the pulse thereafter), the Example was running according to the invention. After depletion of oxygen of $YBa_2Cu_3O_6$ the Example was running as a Comparative Example, i.e. not according to the invention. In Table 5 the compositions of the gases leaving the reactor in the sixth and in the fortieth pulse have been given.

TABLE 4

| Pulse | 6 | 40[1]) |
|---|---|---|
| Gas composition ($\mu$mol) | | |
| isobutane | 31 | 39 |
| isobutene | 45 | 39 |
| hydrogen | 2 | 43 |
| Conversion of isobutane (%)[2]) | 64 | 54 |
| Selectivity to isobutene (%)[3]) | 83 | 85 |
| Hydrogen/isobutene ratio | 0.04 | 1.1 |
| Yield of isobutene (%) | 53 | 46 |

[1]) For comparison, not according to invention

[2]) Intake: 85 $\mu$mol isobutane per pulse

[3]) Defined as 100 x (moles isobutene obtained)/(moles isobutane converted)

Before depletion a conversion of isobutane to isobutene of 64% could be achieved with an isobutene yield of 53%, while the hydrogen/isobutene ratio amounted to 0.05. After depletion the conversion of isobutane to isobutene amounted to 50%, which is practically the dehydrogenation equilibrium conversion at the prevailing reaction conditions, the hydrogen/isobutene ratio being approximately 1.1 and the isobutene yield being 46%. Thus, the Example shows clearly that in the presence of $YBa_2Cu_3O_6$ the dehydrogenation of isobutane can proceed beyond the equilibrium conversion level which results in an improved isobutene yield.

**Claims**

1. A process for the dehydrogenation of organic compounds by contacting a feed containing one or more organic compounds at an elevated temperature with a dehydrogenation catalyst and a perovskite-type oxide which is based upon an oxide of a first metal selected from the Groups 4b, 5b, 6b, 7b, 8 and 1b of the Periodic Table and an oxide of a second metal selected from Group 3b of the Periodic Table.

2. A process according to claim 1, characterized in that the first metal has an atomic number of at least 26.

3. A process according to claim 1 or 2, characterized in that the first metal is copper.

4. A process according to any of claims 1 to 3, characterized in that the second metal is yttrium.

5. A process according to any of claims 1 to 4, characterized in that the perovskite-type oxide contains a third metal selected from Group 2a of the Periodic Table.

6. A process according to claim 5, characterized in that the third metal is calcium, strontium or barium.

7. A process according to claim 6, characterized in that the perovskite-type oxide is selected from the group consisting of $YBa_2Cu_3O_{7-x}$, $Pb_2Sr_2YCu_3O_8$ and $Pb_2Sr_2Ca_{0.5}Y_{0.5}Cu_3O_8$, wherein x is a number from 0 to 1.

8. A process according to any of claims 1 to 7, characterized in that the dehydrogenation catalyst comprises one or more metals selected from the group consisting of vanadium, chromium, molybdenum and the Group 8 noble metals, dispersed on a refractory support.

9. A process according to claim 8, characterized in that the refractory support comprises alumina.

10. A process according to any of claims 1 to 9, characterized in that the feed contains one or more $C_2$ - $C_{10}$ alkanes.

11. A process according to any of claims 1 to 10, characterized in that the dehydrogenation catalyst is operated at a temperature in the range of from 550 to 700 °C, the feed is contacted with the dehydrogenation catalyst during from 0.02 to 2 seconds, the perovskite-type oxide is present at a temperature in the range of from 400 to 700 °C and the contacting time of the feed with the perovskite-type oxide is in the range of from 0.02 to 5 seconds.

12. A process according to any of claims 1 to 11, characterized in that the perovskite-type oxide is treated after the contacting with the feed with molecular oxygen at a temperature in the range of from 750 to 1000 °C during from 2 to 4 hours.

13. A catalyst composition for the dehydrogenation of organic compounds comprising a dehydrogenation catalyst and a perovskite-type oxide which is based upon an oxide of a first metal selected from the Groups 4b, 5b, 6b, 7b, 8 and 1b of the Periodic Table and an oxide of a second metal selected from Group 3b of the Periodic Table.

**PARTIAL EUROPEAN SEARCH REPORT** · Application Number

which under Rule 45 of the European Patent Convention shall be considered, for the purposes of subsequent proceedings, as the European search report

EP    93 20 0528

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | US-A-4 511 754 (GAFFNEY) --- | | C07B35/04 C07C5/42 B01J23/00 |
| A | US-A-4 560 823 (GAFFNEY) ----- | | |

TECHNICAL FIELDS SEARCHED (Int. Cl. 5)

C07C
C07B

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 18 MAY 1993 | J. VAN GEYT |

EPO FORM 1503 03.82 (P04E07)

INCOMPLETE SEARCH

Claims searched completely   : 3-12
Claims searched incompletely : 1-2
Claims not searched          : 13

Reason : Claims 1 and 2 contain vague wordings which make a
         complete search impossible :
         - "organic compounds" has been limited to alkanes
           cf. claim 10)
         - Dehydrogenation catalyst" has been limited to
           catalysts defined in claim 8.
         - "First metal" with an "atomic number of at least
           26" has been limited to copper (cf. claim 3).

         Claim 13 contains the vague wording "dehydrogenation
         catalyst" which makes a meaningful search for the
         catalyst per se impossible.